# EUROPEAN PATENT APPLICATION

(11) **EP 3 425 371 A1**
(43) Date of publication of application: **09.01.2019**
(21) Application number: 17179962.0
(22) Date of filing: 06.07.2017
(51) Int. Cl.: G01N 21/27, G01N 21/71, G01J 3/443

(54) **CALIBRATION OF LASER-INDUCED BREAKDOWN SPECTROSCOPY**

(71) Applicant: Nederlandse Organisatie voor toegepast- natuurwetenschappelijk onderzoek TNO, 2595 DA 's-Gravenhage (NL)
(72) Inventor: van Beijnum, Frerik, 2595 DA's-Gravenhage (NL); Day, James Peter Robert, 2595 DA's-Gravenhage (NL); Klomp, Dolf Jaap, 2595 DA's-Gravenhage (NL)
(74) Representative: V.O.

(57) **Abstract**

Concentrations are determined based on a measurement of a composition (X) by Laser Induced Breakdown Spectroscopy (LIBS). The LIBS spectrum (Sx) comprises resonance peaks (Rk,Rca,Rna) corresponding to the constituents (K,Ca,Na) in the composition (X). The resonance peaks comprise spectral amplitudes (Pk,Pca,Dna,Pna) indicative of the unknown concentrations (Cna,Ck,Cca) of the constituents (K,Ca,Na). A first spectral amplitude (Pk,Pca,Dna) in the LIBS spectrum (Sx) corresponds to the unknown concentration (Ck,Cca,Cna) of a first constituent (K,Ca,Na) to be determined. A second spectral amplitude (Pna) corresponds to a maximum value of a self-reversed resonance peak (Rna) of the first or another constituent (Na) in the LIBS spectrum (Sx). An amplitude ratio (Pk/Pna, Pca/Pna, Dna/Pna) is calculated between the first spectral amplitude (Pk,Pca,Dna) and the second spectral amplitude (Pna) and the ratio is matched with calibration data to determine concentrations.

## Description

### TECHNICAL FIELD AND BACKGROUND

The present disclosure relates to a method and system for determining unknown concentrations of constituents in a composition, in particular based on LIBS measurement. The disclosure also relates to a computer readable medium storing instructions to perform the method and/or used in the system.

In fluid media environments, e.g. in analysis of fluids originating from the human body or of fluids destined for insertion into the human body, such as dialysate, haemodiafiltration fluid or blood serum, but also in plant process environments, in the production of desalinated water etcetera, a desire exists to monitor the fluids' chemical composition or concentrations, in particular of electrolytes and other chemical traces. Common solution for this are in-line conductivity measurement or off-line analytical testing of fluid samples.

With a recent progress of laser technology, compact pulsed lasers are becoming available that combine high beam quality with high pulse energy. When carefully focused, such lasers can deliver energy densities that are strong enough to induce optical breakdown in liquids (e.g. in the order of 10¹⁰ W/cm²). In fluid and other media it is possible to generate a short lived plasma wherein the emission spectrum is indicative for the plasma composition. Spectroscopy techniques of these kinds have been demonstrated in "Laser-induced breakdown spectroscopy (LIBS): "An overview of recent progress and future potential for biomedical applications", Rehse et al, Journal of Medical Engineering & Technology, 2012: 36(20;77-89).

The relative or absolute concentration of constituent elements may be measured by analysing the LIBS spectrum of a composition. Unfortunately, known techniques for calculating concentrations based on LIBS measurements may suffer from poor reproducibility which makes e.g. calibration difficult. Thus it is yet desired to improve accuracy of concentration measurements based on LIBS.

### SUMMARY

According to one aspect, the present disclosure provides a method of determining unknown concentrations of constituents in a composition based on a measurement of the composition by Laser Induced Breakdown Spectroscopy. The method comprises measuring or receiving a LIBS spectrum comprising resonance peaks corresponding to the constituents in the composition. The resonance peaks typically comprise spectral amplitudes indicative of the unknown concentrations of the constituents. A first spectral amplitude in the LIBS spectrum is identified corresponding to the unknown concentration of a first constituent to be determined. A second spectral amplitude is identified corresponding to a characteristic, e.g. maximum, value of a self-reversed resonance peak of the first or another constituents in the LIBS spectrum. The maximum value of the self-reversed resonance peak is typically limited by self-absorption reduction during the LIBS measurement. An amplitude ratio is calculated between the first spectral amplitude and the second spectral amplitude. Calibration data is accessed to match the calculated amplitude ratio with a predetermined calibration amplitude ratio as function of a known concentration of the first constituent. The known concentration of the matched calibration amplitude ratio is used to calculate the unknown concentration of the first constituent in the composition.

The inventors find that the spectral amplitudes of constituents in LIBS spectra may significantly vary between laser pulses even for a fixed concentration. Without being bound by theory, this may be caused by difficult to control changes in the circumstances for each pulse, e.g. pulse energy, plasma variation, etcetera. In any case, the poor reproducibility makes it typically difficult to calibrate the connection between a spectral amplitude and corresponding concentration of a respective constituent. But, the inventors also find that these uncontrolled variations are correlated in particular with the maximum of a self-reversed resonance peak in the LIBS spectrum. At the same time it is found that the maximum of the self-reversed resonance peak is relatively insensitive to concentration variations of the constituent associated with that peak and/or the concentration of another constituent to be measured. Thus by normalizing signals using a ratio of spectral amplitudes, including the maximum of a self-reversed resonance peak, reproducibility of the measurement may be improved.

The concentration of constituents can be derived e.g. taking the ratio between an identified maximum in its associated peak with that of the distinct self-reversed resonance peak, and comparing with calibration ratios at known concentrations. Detailed knowledge of the constituent associated with the self-reversed resonance peak itself may not be necessary in principle as long as the self-reversal is observed. Still, the inventors find that also the concentration of the associated constituent may be derived by correlation with a local dip in the spectrum of the self-reversed resonance peak. Accordingly, a ratio of the dip and maximum within the self-reversed resonance spectrum may be advantageously used to calibrate or compare the concentration of that constituent associated with the self-reversal.

By storing calibration measurements comprising the ratios of spectral amplitudes at a series of known concentrations of one constituent, the ratio of new measurements can be compared to match the concentration of that constituent. For example, the local or overall maximum value in a spectral region associated with the constituent can be used, or a minimum in case of the self-reversed peak. Also other parameters may be derived from the spectrum, e.g. using (linear) decomposition of the LIBS spectrum in components comprising a known or fitted spectral signature of the constituent, if necessary with subtraction of background.

The procedure to derive multiple constituents can e.g. start with the constituent responsible for the self-reversed peak, optionally using that concentration to select calibration data for the ratios of the other constituents. Because the maximum of the self-reversed peak may be relatively insensitive to the concentration association with that peak, it may be sufficient to use calibration data having only approximately the same concentration associated with the self-reversal.

Another factor that may influence the ratio of spectral amplitudes is the light pulse energy. The energy is thus preferably kept as much as possible the same during calibration and actual measurement. The pulse energy may be monitored to optionally select different calibration data. Advantageously, the pulse energy may be derived from the LIBS spectrum, instead of, or in addition to a dedicated sensor. For example, the inventors find that the (white light) background spectrum of a LIBS measurement may be affected by the pulse energy or temperature. Accordingly, e.g. a ratio of intensity of the background spectrum between different wavelengths can be correlated with the pulse energy, e.g. parametrized and/or calibrated.

Calibration data may be stored as an average calibration amplitude ratio based on the average of multiple amplitude ratios. By basing each of the multiple amplitude ratios on the spectral amplitudes of a respective calibration LIBS spectrum, covariation between the amplitudes can be used to improve statistics. For example, a lookup table may store the calibration amplitude ratios and/or analytic descriptions of the calibration amplitude ratio, e.g. as a function of a respective one or more known concentrations of the constituents.

In some aspects, the methods described herein may be embodied as a non-transitory computer readable medium storing instructions that when executed by a computer causes the computer to perform such methods. Another or further aspect provide a LIBS system for determining unknown concentrations of constituents in a composition. Such system may typically comprise one or more of a sample holder configured to hold the composition; a light source and/or optics configured to cause laser induced breakdown in a sample region of the composition; a spectrometer configured to receive and spectrally resolve light from the sample region resulting from the Laser Induced Breakdown; a light sensor configured to measure the spectrally resolved light for determining a LIBS spectrum of the composition; and a controller configured to receive the LIBS spectrum. For example the controller comprises or otherwise has access to a computer readable medium storing instructions to perform one or more methods described herein.

In specific applications, the composition to be analysed may be a biological sample, e.g. blood plasma. For example, typical concentrations of constituents to be determined may include sodium, potassium, and calcium. It is found that for such compositions, e.g. sodium may be present in sufficient concentrations to have an associated self-reversed resonance peak. For example, a kidney dialysis system can make advantageous use of a LIBS system as described herein. The kidney dialysis system may be configured e.g. to monitor constituents directly in a blood plasma and/or in a fluid that is in osmotic contact with the blood plasma, e.g. wherein the blood plasma is circulated to/from a patient.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1A shows an example LIBS spectrum with resonance peaks associated with calcium, sodium and potassium;
FIG 1B shows a zoom-in of the spectrum around the calcium peak;
FIG 1C shows a zoom-in of the spectrum around the sodium peak demonstrating the self-reversal;
FIGs 2A-2C show example calibration data based on ratios of spectral peaks as function of constituent concentrations;
FIGs 3A and 3B illustrate relative insensitivity of calibration data for one constituent to the concentration of another constituent;
FIGs 4A-4C illustrate dependence of calibration data on light pulse energy;
FIG 5A illustrates dependence of background intensities on pulse energy;
FIG 5B shows radiance as a function of wavelength for different temperatures of a black body;
FIGs 6A and 6B illustrates standard deviation as a function of number of measurement points for different quantities;
FIG 7 schematically illustrates a LIBS system;
FIG 8 shows a photograph of a LIBS system.

### DESCRIPTION OF EMBODIMENTS

In some instances, detailed descriptions of well-known devices and methods may be omitted so as not to obscure the description of the present systems and methods. Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. The description of the exemplary embodiments is intended to be read in connection with the accompanying drawings, which are to be considered part of the entire written description. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout.

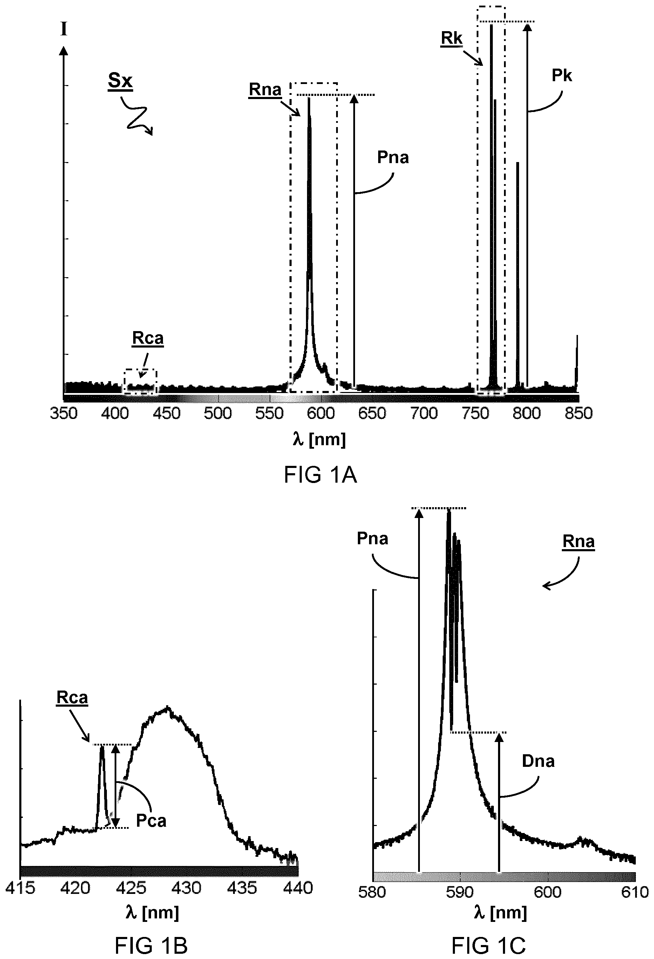

FIG 1A shows an example LIBS spectrum Sx of a composition "X". For example the spectrum Sx can be represented by spectral intensities (I) as function of wavelength λ.

In some embodiments, as in the present example, the composition X comprises a biological sample, e.g. blood plasma. Typically, the concentrations of multiple constituents to be determined may include calcium (Ca), sodium, (Na) and potassium (K), with associated resonance peaks Rca, Rna, Rk, respectively. Various parameters may be derived from the spectrum Sx, e.g. parameters such as peak values Pna, Pk that can be associated with the concentrations of the constituents Na and K in the composition X. Of course other applications may involve other compositions having a different LIBS spectrum e.g. with more or less peaks and/or with different intensity and/or shape. Accordingly, it will be understood that designations as used herein of the spectral resonance peaks, spectral amplitudes, and concentrations linked to specific constituents such as Ca, Na, K are to be considered as exemplary only, for ease of reference, not as limiting to the scope of envisioned embodiments which may involve different constituents with different parameters that can be applied to similar methods mutatis mutandis.

FIG 1B shows a zoom-in of the spectrum around the calcium peak Rca to show that a relatively small peak value Pca can be measured. Optionally peak values may be derived after subtraction of a known, fitted and/or modelled background, in this case a broad background band.

FIG 1C shows a zoom-in of the spectrum around the sodium peak Rna which in this case comprises self-reversed resonance peaks. In other spectra different one or more constituents may exhibit one or more self-reversed peaks. The maximum value of the self-reversed resonance peak is typically limited by self-absorption reduction during the LIBS measurement. Furthermore a self-reversed resonance peak may typically exhibit one or more dips which can be used to define a parameter such as Dna characterizing the intensity at the minimum (or other) dip, e.g. compared to the background intensity or relative to the maximum, e.g. Pna. It is found that this dip may be correlated with the intensity of the constituent forming the self-reversed resonance.

One aspect of the present disclosure provides a method of determining unknown concentrations of respective constituents in a composition based on a measurement of the composition by Laser Induced Breakdown Spectroscopy (LIBS). In one embodiment, the method comprises receiving a LIBS spectrum, e.g. the spectrum Sx as shown in FIG 1A. For example, the LIBS spectrum comprises resonance peaks Rk,Rca,Rna corresponding to the constituents K,Ca,Na in the composition. The resonance peaks typically comprise spectral amplitudes such as Pk,Pca,Dna,Pna indicative of the unknown concentrations Cna,Ck,Cca of the constituents K,Ca,Na.

Preferably, the method comprises determining a first spectral amplitude Pk,Pca,Dna in the LIBS spectrum Sx corresponding to the unknown concentration Ck,Cca,Cna of a first constituent K,Ca,Na to be determined. A second spectral amplitude Pna is determined corresponding to a maximum value of a self-reversed resonance peak Rna of the first or another constituents Na in the LIBS spectrum Sx. Advantageously the second spectral amplitude can be used for normalizing the first spectral amplitude, e.g. calculating an amplitude ratio Pk/Pna, Pca/Pna, Dna/Pna between the first spectral amplitude Pk,Pca,Dna and the second spectral amplitude Pna.

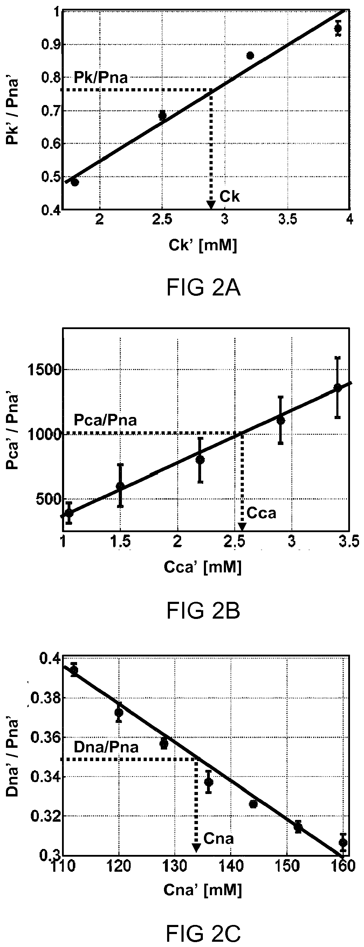

FIGs 2A-2C show example calibration data based on ratios Pk'/Pna', Pca'/Pna', Dna'/Pna' of spectral peaks Pk',Pna',Pca,Dna'. The calibration ratios are recorded as function of known concentrations Ck', Cca, Cna of respective constituents K, Ca, Na.

Some embodiments comprise accessing calibration data to match a calculated amplitude ratio Pk/Pna, Pca/Pna, Dna/Pna of a measured spectrum Sx with a predetermined calibration amplitude ratio Pk'/Pna' Pca'/Pna', Dna'/Pna' as function of a known concentration Ck', Cca', Cna' of one of the constituents K,Ca,Na. The known concentration Ck', Cca', Cna' of the matched calibration amplitude ratio can be used to calculate the unknown concentration Ck, Cca, Cna of said one of the constituents K,Ca,Na in the composition X.

In one embodiment, one of the constituents with unknown concentration to be determined is the same as the constituent corresponding to the self-reversed resonance peak. For example, the first spectral amplitude corresponds to a dip value Dna parametrizing a dip in the self-reversed resonance peak Rna of Na in the LIBS spectrum Sx. In this case the calculated amplitude ratio may be a ratio Dna/Pna between the amplitudes Dna, Pna of a dip and peak value in the same self-reversed resonance peak Rna.

In another or further embodiment, one of the constituents with unknown concentration to be determined is a distinct constituent, e.g. K or Ca, from the constituent corresponding to the self-reversed resonance peak (Na). In this case the first spectral amplitude may correspond e.g. to a peak value Pk,Pca parametrizing a maximum of a resonance peak Rk,Rca in the LIBS spectrum Sx distinct from the self-reversed resonance peak Rna.

Typically, the unknown concentrations Cna,Ck,Cca of multiple constituents are determined including one constituent such as Na corresponding to the self-reversed resonance peak Rna, and a further one or more constituents such as K, Ca corresponding to another one or more resonance peaks Rk, Rca in the LIBS spectrum Sx distinct from the self-reversed resonance peak Rna.

In one embodiment, the calibration amplitude ratio is based on previous calibration measurements with LIBS spectra comprising corresponding first and second spectral amplitudes at a series of known concentrations Ck',Cca',Cna' of the first constituent K,Ca,Na. Preferably a concentration Cna' of the constituent Na corresponding to the self-reversed resonance peak Rna is sufficiently high to exhibit self-absorption reduction during the LIBS measurement of the calibration measurements.

In some embodiments, a spectral amplitude corresponding to a constituent is calculated based on a local or overall maximum value of the LIBS spectrum Sx in a spectral region associated with the constituent. In some embodiments, a spectral amplitude corresponding to a constituent is calculated based on a local or overall minimum value of the LIBS spectrum Sx in a self-reversed resonance peak Rna associated with the constituent. In other or further embodiments, a spectral amplitude corresponding to a constituent is calculated by linear decomposition of the LIBS spectrum Sx in components comprising a spectral signature of the constituent. Also other techniques may be used to extract relevant parameters from the LIBS spectrum.

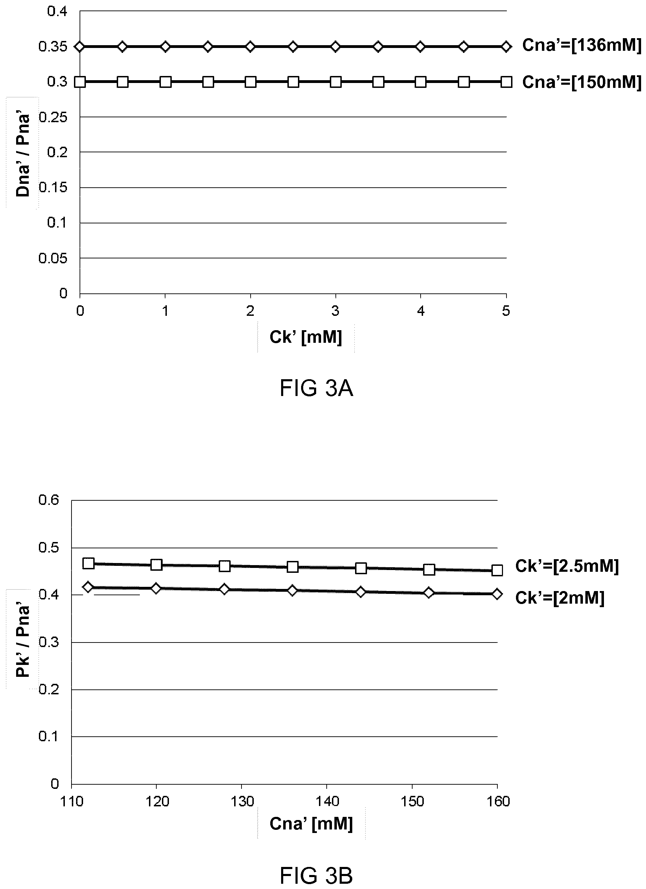

FIGs 3A and 3B illustrate relative insensitivity of calibration data for one constituent to the concentration of another constituent. FIG 3A shows the ratio Dna'/Pna' between the dip and maximum values in the self-reversed resonance peak Rna. The ratio is different for different concentration Cna' of the constituent Na as was also shown in FIG 2C, e.g. lower for higher concentrations Cna'. On the other hand, the ratio has no discernible dependence on the concentration Ck' of potassium (K) over the shown range up to five millimolar. FIG 3B shows the ratio Pk'/Pna' between the peak of the potassium resonance Rk and the maximum of the self-reversed resonance peak Rna. As is shown there is a rather strong dependence on the potassium concentration Ck', but little dependence on the sodium concentration Cna'.

In one embodiment, the concentration Cna of a first constituent Na is determined by matching a ratio of the dip and peak values Dna/Pna of the self-reversed resonance peak Rna with a corresponding ratio Dna'/Pna' in the calibration data. In a further embodiment, the concentration Cna of the first constituent Na, calculated on the basis thereof, is used in subsequently selecting calibration data for determining a concentration of another constituent e.g. K, Ca.

In some embodiments, the calibration data used to determine the unknown concentration of a second constituent, e.g. K, Ca, i.e. other than a first constituent corresponding to the self-reversed resonance peak (Rna) is based on previous measurements with similar or matching concentrations of the first constituent, e.g. Na, corresponding to the self-reversed resonance peak. For example, the said concentrations Cna determining the calibration and actual measurement data differ no more than a factor two, preferably less than one-and-half, or less than one-and-a-quarter, or using calibration data with as close as possible the same concentration of the constituent Na corresponding to the self-reversed resonance peak Rna, or interpolating between available calibration data at nearby concentrations Cna.

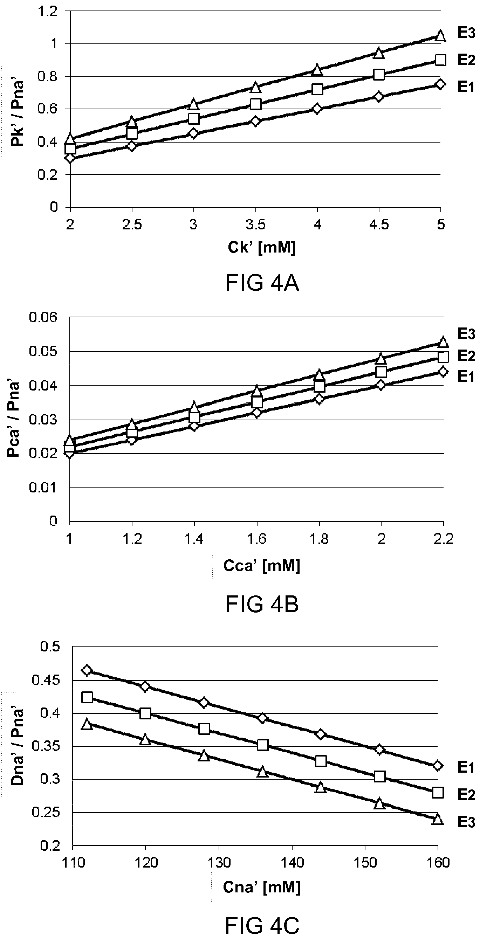

FIGs 4A-4C illustrate possible dependences of the calibration amplitude ratios Pk'/Pna' Pca'/Pna', Dna'/Pna' s on different light pulse energy E1,E2,E3. In one embodiment, the calibration data used to match the calculated amplitude ratio is based on previous measurements using the same or similar light pulse energy as the measurement to determine the unknown concentrations Ck,Cca,Cna. For example, the pulse energy is the same within ten percent, preferably within five percent, more preferably within one percent, or as close as possible having the same pulse energy. Alternatively, or additionally, it is preferred to keep the pulse energy for multiple points in one measurement as constant as possible, e.g. within similar margins, preferably with a pulse-to-pulse energy variation of less than one percent, or better.

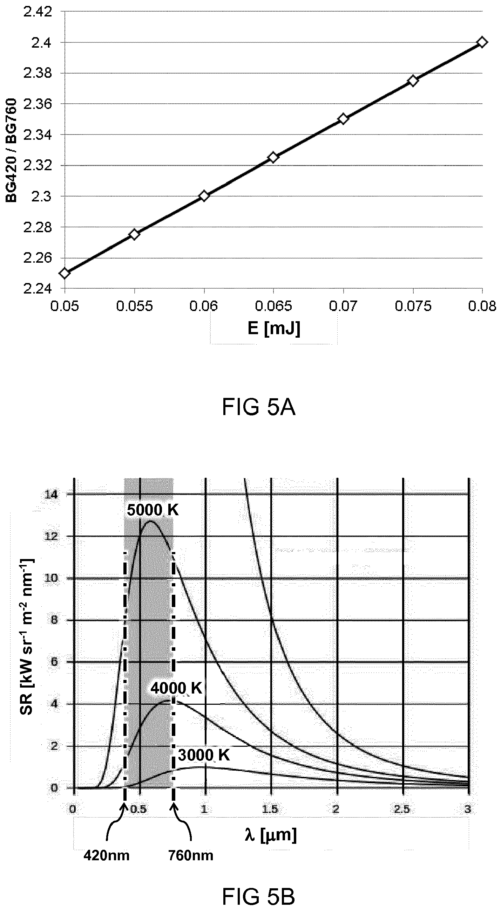

FIG 5A illustrates dependence of background intensities on pulse energy E. For example, a ratio of background intensities BG240/BG760 is taken at respective wavelengths of 420 nm and 760 nm. For higher pulse energies E, the ratio increase by a shift of the background spectrum to towards lower wavelengths. FIG 5B shows expected distributions of spectral radiance SR as a function of wavelength λ for different temperatures of a black body. The positions of possible background measurements at 420 nm and 760 nm are also shown. This illustrates the shift of the distribution towards lower wavelengths, which may be an at least qualitative model for the temperature dependent background radiation of a LIBS measurement.

In one embodiment, an indication of light pulse energy E of a laser pulse, used during measurement to generate the LIBS spectrum Sx, or temperature of the composition X during measurement, is calculated based on a comparison of spectral background intensities at different wavelengths e.g. BG240/BG760 of the LIBS spectrum Sx. In another or further embodiment, the spectral background intensities are measured at wavelengths distinct from the resonance peaks Rk,Rca,Rna, e.g. a white light background.

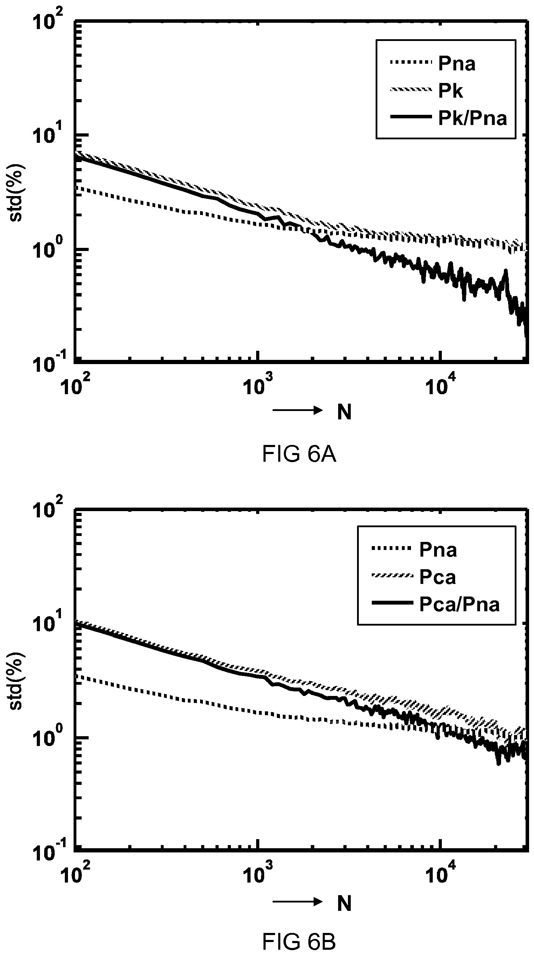

FIGs 6A and 6B illustrate standard deviation "std" as a function of number of measurement points N for different spectral parameters and combinations thereof.

In one embodiment, the first spectral amplitude, e.g. Pk,Pca,Dna, in a calibration ratio is correlated to, or covariant with, the second spectral amplitude, e.g. Pna. For example, a standard deviation std in an average of multiple N consecutive measurements of the first and second spectral amplitudes, is lower for the amplitude ratio between the first and second spectral amplitudes Pk/Pna, Pca/Pna than the first spectral amplitudes Pk,Pca, and/or the second spectral amplitude Pna individually.

In some embodiments, the calibration data is thus stored as an average calibration amplitude ratio Pk'/Pna' Pca'/Pna', Dna'/Pna' based on the average of multiple amplitude ratios. In other or further embodiments, each of the multiple amplitude ratios is based on the first and second amplitudes of a respective calibration LIBS spectrum. It will be appreciated that by first calculating the ratios and then averaging, a better accuracy may be obtained than e.g. storing the average amplitudes Pk', Pna', Pca', and taking the ratio of the averages afterwards. In one embodiment, the calibration data are stored as calibration amplitude ratios in a lookup table, e.g. as a function of a respective one or more known concentrations of the constituents. In another or further embodiment, the calibration data comprises analytic descriptions of the calibration amplitude ratio, e.g. as a function of a respective one or more known concentrations of the constituents.

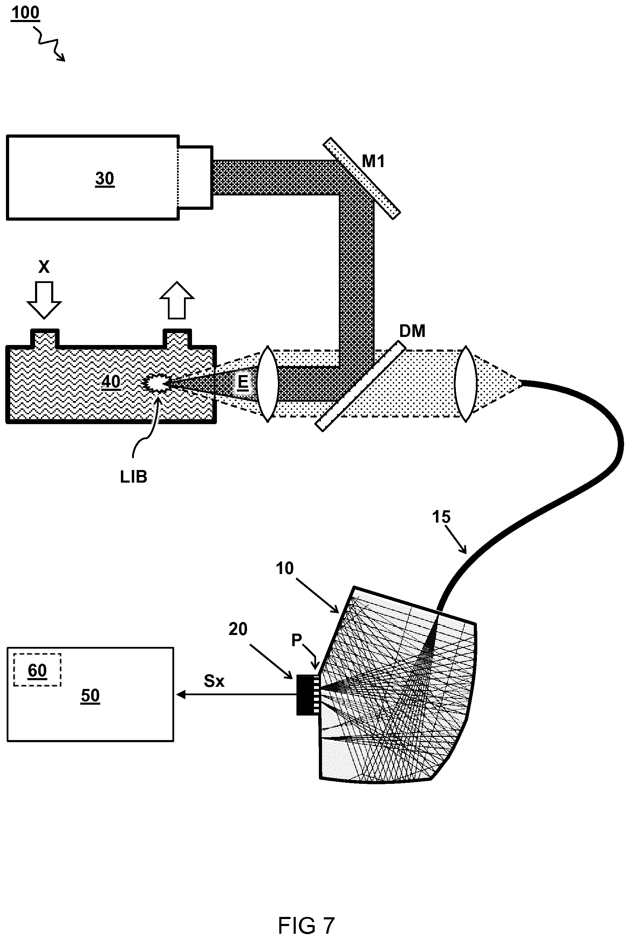

FIG 7 schematically illustrates an example embodiment of a LIBS system 100 for determining unknown concentrations of constituents in a composition X.

In one embodiment, the system 100 comprises a sample holder 40 configured to hold the composition X. For example the composition can be a liquid that is optionally flowed through a sample cell. Also other states of matter can be investigated using LIBS, e.g. gas or even solid. In another or further embodiment, the system 100 comprises or couples to a light source 30 and/or further optics configured to cause laser induced breakdown (LIB) in a sample region of the composition X. The present figure shows a mirror M1 and dichroic mirror DM to guide the light beam from the laser to the sample. Lenses and/or curved mirrors may be used to focus the beam in the sample region and/or collect the resulting light from the region. The dichroic mirror DM may also separate the source laser light from the light caused by LIB.

In one embodiment, a spectrometer 10 is configured to receive and spectrally resolve light from the sample region resulting from the LIB. For example, light is coupled into the spectrometer via an optical fibre 15, or otherwise. In some embodiments, the spectrometer may include or output to a light sensor 20 configured to measure the spectrally resolved light for determining a LIBS spectrum Sx of the composition X. In one embodiment, a monolithic spectrometer is used to obtain a compact and reliable setup.

In one embodiment, a controller 50 is configured to receive the LIBS spectrum Sx. The controller may comprise a computer readable medium 60 that causes it to perform methods for calculating concentrations as described. Some aspects of the present disclosure may relate a non-transitory computer readable medium 60 storing instructions that when executed by a computer causes the computer to perform methods as described herein.

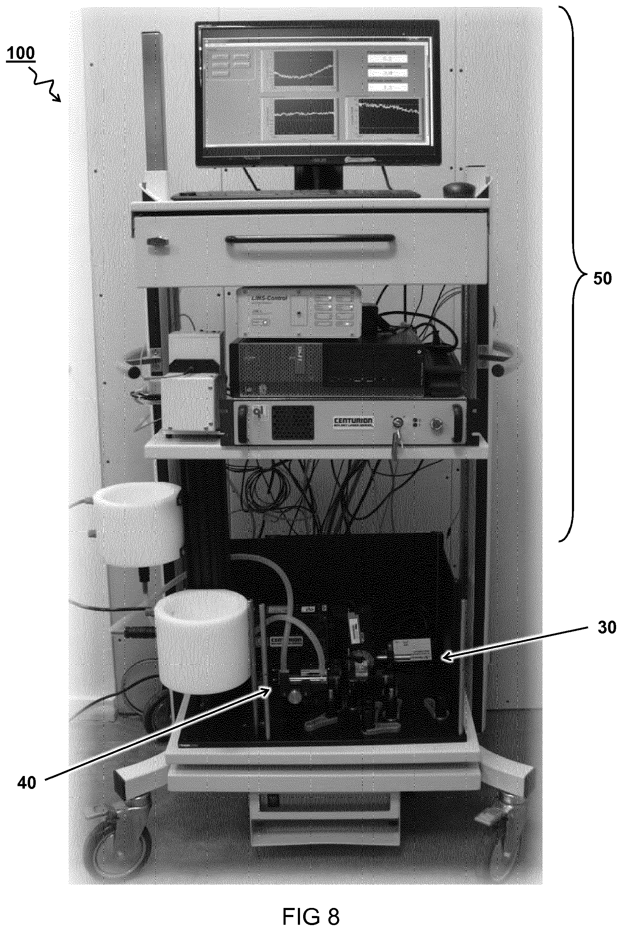

FIG 8 shows a photograph of a demonstrator embodiment for a LIBS system which may find application in a kidney dialysis system. The light source 30, sample holder 40 and controller 50 are roughly indicated. In one embodiment, a kidney dialysis system is configured to monitor constituents such as K, Ca, Na ions directly in a blood plasma and/or in a fluid that is in osmotic contact with the blood plasma. For example, the blood plasma is circulated to/from a patient.

For the purpose of clarity and a concise description, features are described herein as part of the same or separate embodiments, however, it will be appreciated that the scope of the invention may include embodiments having combinations of all or some of the features described. For example, while embodiments were shown for particular parameters and combinations thereof, also alternative ways may be envisaged by those skilled in the art having the benefit of the present disclosure for achieving a similar function and result. E.g. parameters may be further combined or processed, e.g. scaled, inverted, used as input for another function, etcetera, resulting in the same or similar information content. The various elements of the embodiments as discussed and shown offer certain advantages, such as improved accuracy LIBS calibration. Of course, it is to be appreciated that any one of the above embodiments or processes may be combined with one or more other embodiments or processes to provide even further improvements in finding and matching designs and advantages. It is appreciated that this disclosure offers particular advantages to the analysis of biological samples involving known constituents such as Ca, Na, K, at unknown concentrations, and in general can be applied for any application wherein LIBS is used as a quantitative tool.

Finally, the above-discussion is intended to be merely illustrative of the present systems and/or methods and should not be construed as limiting the appended claims to any particular embodiment or group of embodiments. The specification and drawings are accordingly to be regarded in an illustrative manner and are not intended to limit the scope of the appended claims. In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. The mere fact that certain measures are recited in mutually different claims does not indicate that a combination of these measures cannot be used to advantage. In particular, all working combinations of the claims are considered inherently disclosed.

## Claims

1. A method of determining unknown concentrations (Cna,Ck,Cca) of constituents (K,Ca,Na) in a composition (X) based on a measurement of the composition (X) by Laser Induced Breakdown Spectroscopy (LIBS), the method comprising
- receiving a LIBS spectrum (Sx) comprising resonance peaks (Rk,Rca,Rna) corresponding to the constituents (K,Ca,Na) in the composition (X), the resonance peaks comprising spectral amplitudes (Pk,Pca,Dna,Pna) indicative of the unknown concentrations (Cna,Ck,Cca) of the constituents (K,Ca,Na);
- determining a first spectral amplitude (Pk,Pca,Dna) in the LIBS spectrum (Sx) corresponding to the unknown concentration (Ck,Cca,Cna) of a first constituent (K,Ca,Na) to be determined;
- determining a second spectral amplitude (Pna) corresponding to a maximum value of a self-reversed resonance peak (Rna) of the first or another constituents (Na) in the LIBS spectrum (Sx), wherein the maximum value of the self-reversed resonance peak (Rna) is limited by self-absorption reduction during the LIBS measurement;
- calculating an amplitude ratio (Pk/Pna, Pca/Pna, Dna/Pna) between the first spectral amplitude (Pk,Pca,Dna) and the second spectral amplitude (Pna);
- accessing calibration data to match the calculated amplitude ratio (Pk/Pna, Pca/Pna, Dna/Pna) with a predetermined calibration amplitude ratio (Pk'/Pna' Pca'/Pna', Dna'/Pna') as function of a known concentration (Ck',Cca',Cna') of the first constituent (K,Ca,Na); and
- using the known concentration (Ck',Cca',Cna') of the matched calibration amplitude ratio to calculate the unknown concentration (Ck,Cca,Cna) of the first constituent (K,Ca,Na) in the composition (X).

2. The method according to claim 1, wherein one of the constituents with unknown concentration to be determined is the same as the constituent (Na) corresponding to the self-reversed resonance peak (Rna), wherein the first spectral amplitude corresponds to a dip value (Dna) parametrizing a dip in the self-reversed resonance peak (Rna) in the LIBS spectrum (Sx).

3. The method according to claim 1 or 2, wherein one of the constituents with unknown concentration to be determined is a distinct constituent (K,Ca) from the constituent (Na) corresponding to the self-reversed resonance peak (Rna), wherein the first spectral amplitude corresponds to a peak value (Pk,Pca) parametrizing a maximum of a resonance peak (Rk,Rca) in the LIBS spectrum (Sx) distinct from the self-reversed resonance peak (Rna).

4. The method according to any of the preceding claims, wherein the unknown concentrations (Cna,Ck,Cca) of multiple constituents are determined including one constituent (Na) corresponding to the self-reversed resonance peak (Rna), and a further one or more constituents (K,Ca) corresponding to another one or more resonance peaks (Rk,Rca) in the LIBS spectrum (Sx) distinct from the self-reversed resonance peak (Rna).

5. The method according to any of the preceding claims, wherein the concentration (Cna) of the first constituent (Na) is determined by matching a ratio of the dip and peak values (Dna/Pna) of the self-reversed resonance peak (Rna) with a corresponding ratio (Dna'/Pna') in the calibration data, wherein the concentration (Cna) of the first constituent (Na) calculated on the basis thereof is used in subsequently selecting calibration data for determining a concentration of another constituent (K,Ca).

6. The method according to any of the preceding claims, wherein the calibration amplitude ratio is based on previous calibration measurements with LIBS spectra comprising corresponding first and second spectral amplitudes at a series of known concentrations (Ck',Cca',Cna') of the first constituent (K,Ca,Na), wherein a concentration (Cna') of the constituent (Na) corresponding to the self-reversed resonance peak (Rna) is sufficiently high to exhibit self-absorption reduction during the LIBS measurement of the calibration measurements.

7. The method according to any of the preceding claims, wherein the calibration data used to determine the unknown concentration of constituents other than the constituent corresponding to the self-reversed resonance peak (Rna) is based on previous measurements with similar or matching concentrations (Cna) of the constituent (Na) corresponding to the self-reversed resonance peak (Rna), wherein the said concentrations (Cna) determining the calibration and actual measurement data differ no more than a factor two.

8. The method according to any of the preceding claims, wherein the calibration data used to match the calculated amplitude ratio is based on previous measurements using the same light pulse energy (E) as the measurement to determine the unknown concentrations (Ck,Cca,Cna), wherein the pulse energy (E) is the same within one percent.

9. The method according to any of the preceding claims, wherein an indication of light pulse energy (E) of a laser pulse, used during measurement to generate the LIBS spectrum (Sx), or temperature of the composition (X) during measurement, is calculated based on a comparison of spectral background intensities at different wavelengths (BG240/BG760) of the LIBS spectrum (Sx).

10. The method according to any of the preceding claims, wherein the first spectral amplitude (Pk,Pca,Dna) is correlated to, or covariant with, the second spectral amplitude (Pna), wherein a standard deviation (std) in an average of multiple (N) consecutive measurements of the first and second spectral amplitudes, is lower for the amplitude ratio between the first and second spectral amplitudes (Pk/Pna, Pca/Pna) than the first spectral amplitudes (Pk,Pca) by itself.

11. The method according to any of the preceding claims, wherein the calibration data is stored as an average calibration amplitude ratio (Pk'/Pna' Pca'/Pna', Dna'/Pna') based on the average of multiple amplitude ratios, wherein each of the multiple amplitude ratios is based on the first and second amplitudes of a respective calibration LIBS spectrum.

12. The method according to any of the preceding claims, wherein the composition (X) comprises a biological sample, wherein the concentrations of multiple constituents are determined including sodium (Na), potassium (K), and calcium (Ca), wherein the constituent corresponding to the self-reversed resonance peak (Rna) is sodium (Na).

13. A non-transitory computer readable medium storing instructions that when executed by a computer causes the computer to perform the method according to any of the preceding claims.

14. A LIBS system (100) for determining unknown concentrations (Cna,Ck,Cca) of constituents (K,Ca,Na) in a composition (X), the system comprising
- a sample holder (40) configured to hold the composition (X);
- a light source (30) and/or optics configured to cause Laser Induced Breakdown (LIB) in a sample region of the composition (X);
- a spectrometer (10) configured to receive and spectrally resolve light from the sample region resulting from the Laser Induced Breakdown (LIB);
- a light sensor (20) configured to measure the spectrally resolved light for determining a LIBS spectrum (Sx) of the composition (X); and
- a controller (50) configured to receive the LIBS spectrum (Sx),
wherein the controller comprises the computer readable medium of claim 13.

15. A kidney dialysis system (800) comprising the LIBS system (100) according to claim 14.
